# EUROPEAN PATENT APPLICATION

(11) **EP 0 699 749 A1**
(43) Date of publication of application: **06.03.1996**
(21) Application number: 95113645.6
(22) Date of filing: 30.08.1995
(51) Int. Cl.: C12N 9/10, C12N 15/52, C08B 37/16, C12P 19/04

(54) **Recombinant cycloisomaltooligosaccharide synthase, DNA encoding it, and its use**

(30) Priority: 30.08.1994 JP 205631/94
(71) Applicant: NODA INSTITUTE FOR SCIENTIFIC RESEARCH, Noda-shi, Chiba-ken 278 (JP); KIKKOMAN CORPORATION, Noda-shi, Chiba-ken 278 (JP)
(72) Inventor: Oguma, Tetsuya, c/o Noda Inst. for Scientific Res., Noda-shi, Chiba-ken 278 (JP); Kurokawa, Toshiko, c/o Noda Inst. for Scientific, Noda-shi, Chiba-ken 278 (JP); Tobe, Koichiro, c/o Kikkoman Corporation, Noda-shi, Chiba-ken 278 (JP)
(74) Representative: VOSSIUS & PARTNER

(57) **Abstract**

The present invention describes DNA molecules coding for cycloisomaltooligosaccharide synthase with a molecule weight of 103 kd with the following restriction enzyme cleavage map; 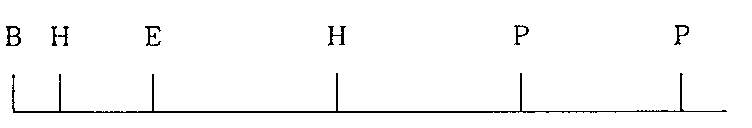
B H E H P P
wherein B stands for Bam HI, E for Eco RI, H for Hind III and P for Pst I.

Furthermore, recombinant DNA molecules are described which contain said DNA inserted into a vector DNA, as well as a process for producing cycloisomaltooligosaccharide synthase by culturing a host cell which comprises said recombinant DNA molecule and which is capable of producing cycloisomaltooligosaccharide synthase in medium and recovering cycloisomaltooligosaccharide synthase from the culture.

According to the present invention, cycloisomaltooligosaccharide synthase can be efficiently obtained for a short time without using expensive dextran in medium.

## Description

The present invention relates to DNA molecules coding for cycloisomaltooligosaccharide synthase, recombinant DNA molecules containing said DNA molecules, and a process for producing cycloisomaltooligosaccharide synthase by culturing hosts carrying said recombinant DNA molecules and recovering cycloisomaltooligosaccharide synthase from the culture.

The cycloisomaltooligosaccharide synthesized from dextran by the action of cycloisomaltooligosaccharide synthase is a novel cyclooligosaccharide which was isolated for the first time by the present inventors from a dextran-containing culture of Bacillus sp. T-3040 isolated from soil. Because this cyclooligosaccharide, similar to cyclodextrin, incorporates a low molecular compound to form an inclusion compound, its use in pharmaceutical preparations, foods, etc., is expected and it is useful as anti cariogenic agent (Japanese Laid-Open Patent Publication No. 197,783/94).

The cycloisomaltooligosaccharide synthase that is an enzyme converting dextran into cycloisomaltooligosaccharide was also isolated for the first time from the culture by the present inventors. This cycloisomaltooligosaccharide synthase is produced by culturing bacteria (e.g. Bacillus sp. T-3040) belonging to the genus Bacillus capable of producing isomaltooligosaccharide synthase in dextran-containing medium and recovering the enzyme from the culture (Japanese Patent Application No. 153,456/93).

However, the above process of producing the enzyme suffers from disadvantages such as a long culture period for Bacillus sp. T-3040, necessity for addition of expensive dextran to medium, and a low yield of the enzyme.

The object of the present invention is to provide DNA molecules coding for cycloisomaltooligosaccharide synthase, recombinant DNA molecules containing said DNA molecules, a process for producing oligosaccharide synthase by culturing hosts carrying said recombinant DNA molecules and recovering cycloisomaltooligosaccharide synthase from the culture.

As a result of their eager study, the present inventors successfully isolated a DNA molecule containing a gene coding for cycloisomaltooligosaccharide synthase from Bacillus sp. T-3040 for the first time. Then, the present inventors determined its sequence and constructed a recombinant DNA molecule by inserting said molecule into a vector DNA. They found that bacteria of the genus Escherichia carrying said recombinant DNA molecule could be cultured to efficiently produce cycloisomaltooligosaccharide synthase.

That is, the present invention relates to DNA molecules coding for a protein with the enzymatic activity of cycloisomaltooligosaccharide synthase with the following restriction enzyme cleavage map: 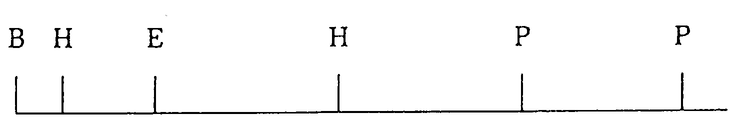
B H E H P P
wherein B stands for Bam HI, E for Eco RI, H for Hind III and P for Pst I.

Further, the present invention relates to DNA molecules coding for cycloisomaltooligosaccharide synthase with the amino acid sequence given in Sequence No. 1, DNA molecules with the nucleotide sequence given in Sequence No. 2 coding for cycloisomaltooligosaccharide synthase, and DNA molecules coding for mature cycloisomaltooligosaccharide synthase with the amino acid sequence given in Sequence No. 4.

The present invention also relates to DNA molecules which hybridise to the above described DNA molecules and which encode cycloisomaltooligosaccharide synthase. The term "hybridisation" in this context means hybridisation under conventional hybridisation conditions, preferably under stringent conditions such as described, for example, by Sambrook et al. (1989, Molecular Cloning, A Laboratory Manual, 2nd Edition, Cold Spring Harbor Laboratory Press, Cold Spring Harbor, NY).
These DNA molecules which hybridise to the above described DNA molecules according to the invention also encompass fragments of these DNA molecules which encode a cycloisomaltooligosaccharide synthase as well as derivatives and allelic variations which differ from the above described molecules due to the alteration of the DNA sequence at one or more positions. These alterations include, for example, deletions, substitutions, recombinations and insertions.

Furthermore, the present invention relates to DNA molecules encoding a cycloisomaltooligosaccharide synthase the DNA sequences of which are degenerate in comparison to the DNA sequences of the above-described DNA molecules due to the degeneracy of the genetic code.

In a preferred embodiment of the present invention the cycloisomaltooligosaccharide synthase encoded by the above described DNA molecules has a molecular weight of approximately 103 kD.

In a further preferred embodiment the DNA molecule encoding cycloisomaltooligosaccharide synthase is derived from a bacterium of the genus Bacillus.

Further, the present invention relates to recombinant DNA molecules, preferably vectors, comprising any one of the above described DNA molecules (excluding the DNA coding for mature cycloisomaltooligosaccharide synthase with the amino acid sequence of Sequence No. 4).

In a preferred embodiment of the present invention the DNA molecule present in the vector is combined with regulatory DNA elements which ensure the expression of the cycloisomaltooligosaccharide synthase in procaryotic or eucaryotic cells.

Furthermore, the present invention relates to host cells comprising a recombinant DNA molecule or vector as described above. The host cell may be a bacterial, fungal, plant or animal cell. In a preferred embodiment the host cell is a bacterial cell, preferably of the genus Escherichia, and most preferably of the species Escherichia coli.

Furthermore, the present invention relates to a process for producing cycloisomaltooligosaccharide synthase by culturing a host cell as described above, preferably a cell belonging to the genus Escherichia, in a medium and recovering cycloisomaltooligosaccharide synthase from the culture.

The present invention also relates to proteins with the enzymatic activity of a cycloisomaltooligosaccharide synthase encoded by a DNA molecule according to the invention.

In another aspect the present invention relates to the use of the cycloisomaltooligosaccharide synthase encoded by the above described DNA molecules for the production of cycloisomaltooligosaccharides, as well as to the cycloisomaltooligosaccharides obtainable by the conversion of α-1,6 glucans, preferably dextran, into cycloisomaltooligosaccharides with the help of the above described enzyme.

First, the preparation of the DNA comprising a gene coding for cycloisomaltooligosaccharide synthase is described.

The donor organism carrying the gene coding for the enzyme may be any one insofar as it produces the enzyme. Examples of such organisms include bacteria of the genus Bacillus, for example, Bacillus sp. t-3040 (FERM BP-4132) etc. The method of culturing such organisms is essentially the same as that used in aerobic culture for conventional organisms, and shake culture or spinner culture under aeration in liquid medium is usually used. Use is made of a medium containing suitable nitrogen and carbon sources, vitamins, minerals and dextran (i.e. an inducing substrate for the present enzyme). The pH of the medium is preferably in the range of 6 to 8, but the pH value may be in any range in which the microorganism can grow.

Shake culture or spinner culture under aeration is carried out usually at 20 to 40°C from 16 hours to 4 days. The culture thus obtained is centrifuged at 3,000 r.p.m. or more, preferably 8,000 to 10,000 r.p.m., for 5 min or more, preferably 10 to 15 min. Genomic DNA from this microorganism can be obtained by a method such as described by Saito and Miura in Biochem. Biophys. Acta, 72, 619 (1963).

Then, the genomic DNA is partially digested with a restriction enzyme such as Sau 3A1 (Boehringer Mannheim) for 20 min or more, preferably 30 min to 2 hours, at a temperature of 30 °C or more, preferably 37 °C, at an enzyme concentration of 0.1 to 10 U/ml, to generate protruding ends. The resulting genomic DNA fragments of various chain lengths may be purified if necessary by purification procedures known in the art, including extraction with phenol, extraction with phenol and chloroform, and precipitation with ethanol.

The vector DNA used in the present invention may be any kind of vector DNA including plasmid vector DNA, bacteriophage vector DNA, etc. A preferable example is plasmid pUC118 DNA (Takara Shuzo Co., Ltd.).

To generate protruding ends, the vector DNA is partially digested with a restriction enzyme such as Bam HI or the like for 1 hour or more, preferably 1 to 3 hours, at a temperature of 30 °C or more, preferably 37 °C, at an enzyme concentration of 10 to 1000 U/ml. The cleaved vector DNA may be purified if necessary by purification procedures known in the art, including extraction with phenol, extraction with phenol and chloroform, and precipitation with ethanol.

A recombinant DNA is obtained by ligating said cleaved genomic DNA into said cleaved vector DNA by the action of E. coli DNA ligase (New England Biolabs), T4DNA ligase (Boehringer Mannheim), etc., preferably T4DNA ligase, for 1 hour or more, preferably 6 to 24 hours, at a temperature of 4 to 37°C, preferably 4 to 16°C, at an enzyme concentration of 1 to 100 U/ml. In this step, a ligation kit commercially available from Takara Shuzo Co., Ltd. may also be used.

This recombinant DNA is used to transform or transduce host cells, preferably bacteria such as E. coli K-12, preferably E. coli JM101 (ATCC 33876), E. coli JM109 (Takara Shuzo Co., Ltd.), E. coli HB101 (ATTCC 33694), E. coli DH I (ATCC 33849), E. coli χ-1776 (ATCC 31244), E. coli XL1-Blue MRF' (Toyobo Co., Ltd.) etc. Transformation can be effected by the D.M. Morrison method in Methods in Enzymology, 68, 326-331 (1979) and transduction by the B. Hohn method in Methods in Enzymology, 68, 299-309 (1979).

A strain belonging to the genus Escherichia carrying the recombinant DNA comprising a gene coding for cycloisomaltooligosaccharide synthase inserted into a vector DNA can be obtained by screening for a microorganism having the ability to produce cycloisomaltooligosaccharide synthase.

The novel purified recombinant DNA can be obtained from the strain as described by P. Guerry et al. in J. Bacteriology, 116, 1064-1066 (1973) or D. B. Clewell in J. Bacteriology, 110, 667-676 (1972).

The DNA comprising a gene coding for cycloisomaltooligosaccharide synthase is used for sequencing of the whole nucleotide sequence of the gene (Sequence No. 2) by the Sanger/dideoxy termination method using Taq polymerase as illustrated below in item (4) in Example 1, and the primary sequence of the polypeptide encoded thereby is determined (Sequence No. 1).

The method of culturing the resulting strain carrying the recombinant DNA to produce cycloisomaltooligosaccharide synthase is essentially the same as that used in aerobic culture for conventional bacteria, and shake culture or spinner culture under aeration in liquid medium is usually preferable. The medium used is prepared by adding one or more nitrogen sources such as yeast extract, peptone, meat extract, corn steep liquor and exudate of soybean or wheat bran, one or more inorganic salts such as potassium dihydrogen phosphate, dipotassium hydrogen phosphate, magnesium sulfate, sodium chloride, magnesium chloride, ferric chloride, ferric sulfate and manganese sulfate, and if necessary, sugars or carbohydrates and vitamins. The initial pH value is adjusted preferably within the range of 7 to 9. Culture is continued for 4 to 24 hours, preferably 6 to 24 hours, at a temperature of 30 to 42 °C, preferably around 37°C.

After culture is concluded, the cycloisomaltooligosaccharide synthase can be recovered using enzyme recovery means known in the art.

For example, a crude enzyme preparation is obtained as follows:
The enzyme is extracted from the organism by disrupting the organism by ultrasonication or grinding, extracting the enzyme with lytic enzyme such as lysozyme, or autolysis in the presence of solvent such as toluene optionally with shaking. After insolubles such as cell debris are removed by filtration or centrifugation optionally followed by removal of nucleic acids by precipitation with streptomycin sulfate, protamine sulfate or manganese sulfate, the lytic solution is fractionated with ammonium sulfate, alcohol, acetone or the like, and the precipitate is recovered, dialyzed against water and dried under vacuum.

This preparation of crude cycloisomaltooligosaccharide synthase can be further purified by a suitable combination of chromatographic procedures including ion-exchange chromatography on DEAE-Sepharose (diethylethylamine-Sepharose, Pharmacia), DEAE-Sephadex (Pharmacia), etc. and gel filtration on Sephadex G-200 (Pharmacia), Bio-Rad P-150 (Bio-Rad), etc., if necessary followed by a suitable combination of HPLC (high performance liquid chromatography) including ion-exchange chromatography on TSKgel DEAE-5PW (Tohso Co., Ltd.) etc. or hydrophobic chromatography on TSKgel ether 5PW (Tohso), TSKgel phenyl 5PW (Tohso), etc., or gel filtration on TSKgel G3000SW (Tohso) etc.

The physicochemical properties of the cycloisomaltooligosaccharide synthase thus purified are as follows:

### (1) Action

The enzyme acts on a polymer of glucoses bound via α-1,6 linkages such as dextran etc. to produce cycloisomaltooligosaccharides by intramolecular glycosyltransfer reaction.

### (2) Substrate specificity

The enzyme acts on dextran having an α-1,6 linkage as the main chain, but not on aminopectin, pullulan, etc., having an α-1,6 linkage of glucoses partially in the molecule.

### (3) Optimum pH and pH stability

The enzyme acts in the vicinity of pH 5.5 and is stable in the range of pH 4.5 to 8.5.

The isomaltooligosaccharide synthase can be efficiently produced for a short period without using expensive dextran in medium by the use of a strain belonging to the genus Escherichia carrying a recombinant DNA containing a cycloisomaltooligosaccharide synthase gene.

The present invention is described in more detail with reference to the following examples, which however are not intended to limit the scope of the present invention.

### Example 1

### Isolation and Sequencing of Cycloisomaltooligosaccharide Synthase Gene

### (1) Preparation of genomic DNA from Bacillus sp. T-3040

100 ml liquid medium (tap water, pH 7.1) containing 1 % dextran 40 (Meito Sangyo Co., Ltd.), 1 % peptone (Kyokuto Seiyaku Co., Ltd.), 0.5 % NaCl and 0.1 % yeast extract (Difco) was introduced into a 500 ml flask and sterilized at 120 °C for 20 min. Bacillus sp. T-3040 (FERM BP-4132) from a slant culture was inoculated into it and cultured at 30 °C for 1 day under shaking. 1 ml of the culture was then inoculated into a large test tube containing 10 ml medium with the same composition sterilized under the same conditions as above and cultured over 2 days at 30°C under shaking at 120 r.p.m. The culture was centrifuged at 800 r.p.m. for 20 min. From about 50 mg wet microorganism, 1 ml genomic DNA solution was obtained according to the method described by Saito and Miura in Biochem. Biophys. Acta, 72, 619 (1963). 360 µl of the genomic DNA solution (about 200 µg genomic DNA) was partially digested at 37 °C for 1 hour with 0.2 unit of restriction enzyme Sau 3AI (Takara Shuzo Co., Ltd.) in 50 mM Tris-HCl buffer, pH 7.4, containing 100 mM NaCl and 10 mM MgSO₄. Then, the DNA fragment was extracted with phenol and precipitated with ethanol. To prevent self-ligation, the fragment was dephosphorylated with calf intestine alkaline phosphatase according to the method described in Molecular Cloning, pp. 133-134, followed by extraction with phenol and precipitation with ethanol, whereby 160 µg Sau 3AI digest of genomic DNA from Bacillus sp. T-3040 was obtained.

### (2) Construction of a genomic DNA library from Bacillus sp. T-3040 in plasmid vector pUC118 DNA and screening for a cycloisomaltooligosaccharide synthase gene

20 µl of plasmid vector pUC118 DNA (Takara Shuzo Co., Ltd.) was digested at 37°C for 2 hours with 10 units of restriction enzyme Bam HI (Takara Shuzo Co., Ltd.) in 50 mM Tris-HCl buffer, pH 7.4 (100 mM NaCl and 10 mM MgSO₄) and then extracted with phenol and precipitated with ethanol to give a Bam HI digest of plasmid vector pUC118.

1 µg of the Bam HI digest of plasmid vector pUC118 and 1 µg of the Sau 3AI digest of genomic DNA from Bacillus sp. T-3040 obtained in item (1) were ligated at 16°C for 16 hours with a DNA ligation kit (Takara Shuzo Co., Ltd.).

100 µl of E. coli competent cells (XL1-Blue MRF') (Toyobo) were transformed with 10 µl of the resulting recombinant plasmid DNA by allowing the cells to stand on ice for 20 min. After transformation, 110 µl of the bacterial suspension was inoculated into 3 ml T-Y medium, pH 7.2 [1 % (W/V) Bacto-trypton (Difco), 0.5 % (W/V) yeast extract (Difco) and 0.5 % (W/V) NaCl] sterilized under the same conditions as above, followed by 30 min incubation at 37°C under shaking. The culture was divided into 200 µl aliquots, and each aliquot was plated onto a sterilized, blue dextran-containing X-gal plate, pH 7.2 in a vessel of 9 cm in diameter [0.5 % (W/V) blue dextran (Pharmacia), 1 % (W/V) Bactotrypton (Difco), 0.5 % (W/V) yeast extract (Difco), 0.5 % (W/V) NaCl and 1.5 % agar sterilized under the same conditions as above, plus 50 µg/ml (W/V) IPTG (isopropyl-β-D-thiogalactopyranoside) (Boehringer Mannheim), 40 µg/ml (W/V) X-gal (5-bromo-4-chloro-3-indolylgalactopyranoside) (Boehringer Mannheim) and 25 µg/ml (W/V) ampicillin (Sigma) sterilized through a filter of 0.22 µm pore (Millpore)]. During 24 hours of stationary culture at 37°C, there occurred colonies among which white colonies were represented as a genomic library. Out of about 2000 colonies in the library, 1 strain was obtained as the positive colony by selecting a colony with a clear halo around it. The positive strain was inoculated into 3 ml T-Y medium containing 50 µg/ml ampicillin and 50 µg/ml IPTG, sterilized under the same conditions as above. After 16 hours of shake culture at 37°C the culture was centrifuged at 8000 r.p.m. for 20 min to give 2 mg wet bacterial precipitate. A plasmid containing the cycloisomaltooligosaccharide synthase gene was extracted from the bacteria with alkali as described in Molecular Cloning, a Laboratory Manual, Second Edition, pp. 25-28, Cold Spring Harbor Laboratory Press (1989), then precipitated with ethanol and dissolved in 200 µl TE buffer, pH 8.0 (10 mM Tris, 1 mM EDTA). This recombinant plasmid DNA was designated pCI429. Then, E. coli XL1-Blue MRF' was transformed with the plasmid DNA by the transformation method described in J. Bacteriology, 119, 1072-1074 (1974) and screened for a transformant with cycloisomaltooligosaccharide synthase activity. The resulting transformant, E. coli XL1-Blue MRF' (pCI429) has been deposited as FERM BP-4783 with the National Institute of Bioscience and Human-Technology, Agency of Industrial Science and Technology, Japan. After 1 µg of this recombinant plasmid DNA was cleaved with a combination of restriction enzymes Hind III (Boehringer Mannheim), Bam HI (Takara Shuzo Co., Ltd.), Eco RI (Boehringer Mannheim) and Pst I (Boehringer Mannheim), the lengths of the DNA fragments were determined by electrophoresis on 5 % agarose gel so that a restriction enzyme cleavage map was prepared.

### (3) Culture of E. coli XL1-Blue MRF' (pCI429) and preparation of a crude enzyme solution

E. coli XL1-Blue MRF' (pCI429) (FERM BP-4783) was cultured in 3 ml T-Y medium for 16 hours under shaking and then disrupted on ice with ultrasonication for 2 min. The cell debris was removed by centrifugation at 12000 r.p.m. for 20 min. The cycloisomaltooligosaccharide synthase activity in the cell extract was about 3 mU/ml. A comparative experiment indicated that there was no cycloisomaltooligosaccharide synthase activity in a cell extract obtained under the same conditions from E. coli JM109 carrying vector DNA pUC118.

### (4) Sequencing of the cycloisomaltooligosaccharide synthase gene derived from Bacillus sp. T-3040 (FERM BP-4132)

10 µg of the recombinant plasmid DNA pCI429 obtained in item (2) was cleaved at 37°C for 1 hour with a mixture of 1 unit of restriction enzyme Sph I (Boehringer Mannheim), 1 unit of restriction enzyme Bam HI (Takara Shuzo Co., Ltd.), 2 µl of a restriction enzyme buffer (Boehringer Mannheim) and 10 µl distilled water. Then, the reaction solution was subjected to 1.5 % agarose gel electrophoresis. The target DNA fragment was extracted from the gel and precipitated with ethanol to give an about 5 µg purified DNA fragment. Separately, 1 µg vector DNA pUC119 was digested at 37°C for 1 hour with a mixture of 1 unit of Sph I (Boehringer Mannheim), 1 unit of Bam HI (Takara Shuzo Co., Ltd.), 2 µl restriction enzyme buffer (Boehringer Mannheim) and 19 µl distilled water. This digest of vector DNA pUC119 was ligated into 2.5 µg of the above purified DNA fragment at 16°C for 16 hours with a ligation kit (Takara Shuzo Co., Ltd.). Then, E. coli XL1-Blue MRF' was transformed with the above reaction solution by the transformation method described in J. Bacteriology, 119, 1072-1074 (1974) and screened for a transformant with cycloisomaltooligosaccharide synthase activity. About 20 µg of a recombinant plasmid DNA was obtained by extracting 10 ml culture of the transformant with alkali as described in Molecular Cloning, a Laboratory Manual, Second Edition, pp. 25-28, Cold Spring Harbor Laboratory Press (1989) and this recombinant plasmid was designated pCI519. The plasmids pCI519 and pCI429 are identical with each other except that the DNA fragment containing the cycloisomaltooligosaccharide synthase gene has been inserted in different directions.

Then, the deleted sequences having various chain length were obtained from the cycloisomaltooligosaccharide synthase gene in the recombinant plasmids pCI429 and pCI519 according to the method described by Henikoff in Gene, 28, 351-359 (1984). E. coli XL1-Blue MRF' (Toyobo) was transformed with the resulting plasmid DNAs in the same manner as in item (2) and then infected with helper phage M13K07 (Takara Shuzo Co., Ltd.), and a single-stranded DNA was prepared according to the method described by Messing in Methods in Enzymology, 101, 20-78 (1983). The single-stranded DNA was sequenced by a DNA sequencer (Applied Biosystem Instrument (ABI)) with a Taq polymerase sequencing kit (Applied Biosystem Instrument (ABI)).

The whole nucleotide sequence of the cycloisomaltooligosaccharide synthase gene derived from Bacillus sp. T-3040 (FERM BP-4132) is shown in Sequence No. 2 and the primary amino acid sequence encoded thereby is shown in Sequence No. 1.

### Example 2

100 ml liquid medium (tap water, pH 7.1) containing 1 % dextran 40 (Meito Sangyo), 1 % peptone (Kyokuto Seiyaku), 0.5 % NaCl and 0.1 % yeast extract (Difco) was introduced into 500 ml flask and sterilized at 120 °C for 20 min. Bacillus sp. T-3040 (FERM BP-4132) from a slant culture was inoculated into it and cultured at 30 °C for 1 day under shaking.

1000 ml of the culture was inoculated into 500 L tank containing 300 L medium with the same composition sterilized under the same conditions as above, followed by 3 days of spinner culture at 30 °C under aeration at 0.25 v.v.m. and 70 r.p.m.. Then, 300 L of the culture was filtered through Microza (a ultrafiltration membrane manufactured by Asahi Kasei Kogyo) to remove the microorganism, and the filtrate was concentrated into a volume of 6.3 L through a follow fiber membrane (cut off molecular weight > 6000). The concentrate was divided into a volume of 900 ml for storage in a freezer at -20 °C.

The cycloisomaltooligosaccharide synthase was purified in the following manner. The frozen concentrate (900 ml) was thawed and dialyzed at 4°C overnight against 10 mM phosphate buffer, pH 7.0, containing 1 mM EDTA. The dialysate was centrifuged to remove insolubles, and the supernatant was applied to a DEAE-Sepharose CL6B column equilibrated with the same buffer. After the column was washed with the same buffer, the adsorbed protein was eluted with a concentration gradient of from 0 to 0.8 M NaCl. The active fractions (320 ml) were combined and ammonium sulfate was added thereto at a final concentration of 1.0 M. The precipitated impurities were removed by centrifugation. The supernatant was further purified by preparative HPLC as follows. The sample was applied to a TSKgel phenyl 5PW column equilibrated with 100 mM phosphate buffer, pH 7.0, containing 1.0 M ammonium sulfate and 10 mM EDTA. The adsorbed protein was washed with the same buffer and eluted with a concentration gradient of from 1.0 to 0 M ammonium sulfate.

The active fractions (150 ml) were combined and ammonium sulfate was added thereto at a final concentration of 1.0 M. The precipitated impurities were removed by centrifugation. The supernatant was applied again to the TSKgel phenyl 5PW column equilibrated with 100 mM phosphate buffer, pH 7.0 containing 1.0 M ammonium sulfate and 10 mM EDTA. The adsorbed protein was washed once with the same buffer except for a decreased ammonium sulfate concentration of 0.3 M and eluted with a concentration gradient of from 0.3 to 0 M ammonium sulfate.

The active fractions (88 ml) were combined and concentrated by ultrafiltration to a volume of about 1 ml, and the salt concentration was decreased by adding 20 ml of 10 mM phosphate buffer, pH 7.0 containing 1 mM EDTA. The solution was applied to a TSKgel DEAE 5PW column equilibrated with 10 mM phosphate buffer, pH 7.0 containing 1 mM EDTA. The adsorbed protein was washed with the same buffer and then with 0.15 M NaCl and eluted with a concentration gradient of from 0.15 to 0.4 M NaCl.

The active fractions (12 ml) were combined and concentrated by ultrafiltration to a volume of 0.9 ml. 0.3 ml of this enzyme concentrate was passed through a TSKgel G3000SW column equilibrated with 100 mM phosphate buffer, pH 7.0 containing 10 mM EDTA and 200 mM NaCl. The remaining enzyme solution (0.6 ml) was divided into 2 aliquots and each aliquot, 0.3 ml, was passed through the column in the same manner. SDS-PAGE showed a single band for the eluted active fraction, indicating the absence of any protein impurities. It was confirmed that dextran was converted into cycloisomaltooligosaccharides by incubating dextran with the purified enzyme. N-terminal amino acid sequence of the polypeptide was determined by a gas-phase-type peptide sequencer (Applied Biosystem Instrument (ABI)). The result is shown in Sequence No. 3.

The primary amino acid sequence of the polypeptide with cycloisomaltooligosaccharide synthase is shown in Sequence No. 4, and it was turned out that a polypeptide adjacent to the N-terminal side of the polypeptide with cycloisomaltooligosaccharide synthase is involved in polypeptide secretion.

SEQUENCE TABLE
Sequence No: 1
Length: 972
Sequence Type: amino acid
Topology: linear
Nature: peptide
Sequence:
Met Val Arg Phe Met Tyr Ala Leu Arg Lys Arg Arg Leu Ser Leu Leu
                5                   10                  15
Leu Ala Met Ser Leu Leu Val Met Cys Val Ala Ser Val Val Ser Pro
            20                  25                  30
Pro Pro Gln Ala Leu Ala Ser Gly Ser Gly Gly Ile Glu Arg Val Phe
        35                  40                  45
Thr Asp Lys Ala Arg Tyr Asn Pro Gly Asp Ala Val Ser Ile Arg Val
    50                  55                  60
Gln Ala Lys Asn Gly Thr Gly Ser Ser Trp Ser Gly Ala Ala Arg Leu
65                  70                  75                  80
Glu Ile Phe His Leu Glu Asn Ser Val Tyr Thr Ser Ser Gln Ser Leu
                85                  90                  95
Ser Leu Thr Asn Gly Gln Ser Thr Thr Leu Thr Phe Thr Trp Thr Ala
            100                 105                 110
Pro Ser Thr Asp Phe Arg Gly Tyr Phe Val Arg Ile Asp Ala Gly Thr
        115                 120                 125
Leu Gly Gln Gly Ala Thr Ala Ile Asp Val Ser Ser Asp Phe Thr Lys
    130                 135                 140
Tyr Pro Arg Tyr Gly Tyr Ile Ser Glu Phe Glu Ser Gly Glu Thr Ala
145                 150                 155                 160
Leu Glu Ser Lys Ala Lys Val Asp Gln Leu Ala Gln Asp Tyr His Ile

                165                 170                 175
Asn Ala Trp Gln Phe Tyr Asp Trp Met Trp Arg His Asp Lys Met Ile
            180                 185                 190
Lys Arg Thr Gly Gly Ser Ile Asp Ser Thr Trp Leu Asp Leu Phe Asn
        195                 200                 205
Arg Glu Ile Ser Trp Ser Thr Leu Gln Asn Gln Ile Asp Ala Val His
    210                 215                 220
Asp Val Asn Gly Lys Ala Met Ala Tyr Ala Met Ile Tyr Ala Ser Arg
225                 230                 235                 240
Glu Asn Tyr Ser Pro Leu Gly Ile Ser Pro Thr Trp Gly Ile Tyr Glu
                245                 250                 255
Asp Ser Ser His Thr Asn Gln Phe Asp Val Asp Phe Gly Asp Gly Ser
            260                 265                 270
Thr Tyr Leu Tyr Met Ser Asp Pro Gln Asn Pro Asn Trp Gln Asn Tyr
        275                 280                 285
Ile His Ala Glu Tyr Ile Asp Ser Ile Asn Thr Ala Gly Phe Asp Gly
    290                 295                 300
Ile His Val Asp Gln Met Gly Gln Arg Ser Asn Val Tyr Asp Tyr Asn
305                 310                 315                 320
Gly Asn Ser Ile Asp Leu Ser Thr Arg Phe Ser Pro Phe Leu Asp Gln
                325                 330                 335
Ala Lys Ser Val Leu Ser Ala Asn Asn Pro Ala Arg Asp Asn Leu Thr
            340                 345                 350
Tyr Asn Ile Val Asp Gly Thr Val Asn Gly Trp Ala Val Asn Asp Val
        355                 360                 365
Ser Lys Asn Ala Asp Leu Asp Phe Leu Tyr Ser Glu Ile Trp Tyr Leu
    370                 375                 380
Ser Asp Ser Tyr Asn Gln Leu Lys Asn Tyr Ile Glu Gln Leu Arg Ala

385                 390                 395                 400
Asn Gly Gly Asn Lys Ala Val Val Leu Ala Ala Tyr Met Asn Tyr Ala
                405                 410                 415
Asp Asn Ala Gly Thr Arg Tyr Glu Ala Glu Ser Ala Ser Met Thr Asn
            420                 425                 430
Val Ser Thr Asn Thr Asn His Ala Gly Tyr Thr Gly Ser Gly Phe Val
        435                 440                 445
Asp Gln Phe Ala Ser Thr Gly Asp Lys Val Ser Phe Ala Ile Asn Ala
    450                 455                 460
Pro Glu Ala Gly Asp Tyr Ser Leu Val Phe Arg Tyr Gly Asn Asn Thr
465                 470                 475                 480
Gly Ala Asn Ser Thr Leu Asn Leu Tyr Val Asp Gly Asn Phe Val Gln
                485                 490                 495
Lys Leu Tyr Phe Phe Asn Gln Ser Ser Trp Gly Thr Trp Lys His Asp
            500                 505                 510
Ala Trp Tyr Gln Val Pro Leu Thr Gln Gly Ala His Thr Val Glu Leu
        515                 520                 525
Arg Tyr Glu Ser Gly Asn Val Gly Ala Val Asn Leu Asp Ser Leu Thr
    530                 535                 540
Leu Gly Thr Phe Asp Glu His Ser Val Arg Leu Ala Asp Ala Met Met
545                 550                 555                 560
Ser Ala Ser Gly Ala Thr His Ile Glu Leu Gly Asp Asp Asn Gln Met
                565                 570                 575
Leu Pro His Glu Tyr Tyr Pro Asn Arg Ser Lys Thr Met Arg Ser Ser
            580                 585                 590
Leu Lys Asn Ala Met Lys Asp His Tyr Asn Phe Ile Thr Ala Tyr Glu
        595                 600                 605
Asn Leu Leu Phe Asp Ser Asp Val Val Pro Asn Asp Thr Gly Ser Gln

610                 615                 620
Phe Val Asn Leu Thr Gly Val Ser Ala Ser Gly Asp Gly Ser Ala Asn
625                 630                 635                 640
Thr Val Trp Tyr Ile Asn Lys Arg Thr Ser Asp Tyr Asn Ile Val His
                645                 650                 655
Leu Ile Asn Leu Leu Gly Asn Asp Asn Gln Trp Arg Asn Thr Ala Ser
            660                 665                 670
Gln Pro Ser Phe Gln Thr Asn Leu Pro Ala Lys Ile Tyr Ile Gly Ala
        675                 680                 685
Asp Glu Thr Ile Ser Asp Val Tyr Leu Ala Ser Pro Asp Leu Ser Gly
    690                 695                 700
Gly Glu Thr Gln Glu Leu Ala Phe Thr Ser Gly Thr Asp Ala Gly Gly
705                 710                 715                 720
Lys Tyr Val Ser Phe Thr Val Pro Glu Leu Lys Tyr Trp Asn Met Ile
                725                 730                 735
Tyr Met Lys Arg Thr Phe Ser Val Pro Ala Asn Asp Ile Tyr Glu Ala
            740                 745                 750
Glu Thr Ala Ile Lys Ser Asn Val Ser Thr Asn Thr Asn His Ala Gly
        755                 760                 765
Tyr Thr Gly Ser Gly Phe Val Asp Gly Phe Ser Ser Thr Asn Asp Gly
    770                 775                 780
Val Ser Phe Val Val Lys Ser Thr Ala Ser Asp Asp Tyr Ala Leu Arg
785                 790                 795                 800
Phe Arg Tyr Ala Asn Gly Gly Ser Asp Ala Thr Arg Asp Val Tyr Val
                805                 810                 815
Asp Gly Lys Leu Ala Gly Thr Val Ser Phe Lys Ser Thr Gly Ser Trp
            820                 825                 830
Ser Thr Trp Ser Tyr Gly Glu Ile Thr Ala Arg Leu Glu Pro Gly His

835                 840                 845
His Thr Ile Val Leu Trp Gln Thr Ser Gly Asn Thr Gly Ala Ile Asn
    850                 855                 860
Leu Asp His Leu Asp Leu Asp Lys Thr Tyr Ile Trp Gln Phe Asp Arg
865                 870                 875                 880
Gln Ile Val Ser Val Pro Ala Gly Tyr Arg Ile Thr Phe Arg Thr Gly
                885                 890                 895
Leu Pro Gly Trp Val His Trp Gly Val Asn Gly Trp Thr Gly Val Thr
            900                 905                 910
Asp Thr Pro Leu Arg Ser Asn Gly Ser Leu Asp Gly Asn Leu Asp His
        915                 920                 925
Glu Thr Ser Ile Gly Pro Phe Ala Thr Gly Thr Ala Val Asp Val Thr
    930                 935                 940
Phe Leu Trp Asp Asp Asn Asn Asn Gly Ile Leu Glu Pro Ser Thr Asp
945                 950                 955                 960
Arg Trp Glu Gly Thr Asp Phe Gly Ile Asn Val Ser
                965                 970

Sequence No:  2
Length:  2916
Nature:  nucleic acid
Strand Form:  single
Topology:  linear
Nature:  genomic DNA
Sequence:
ATGGTGAGAT TTATGTACGC TTTAAGGAAA AGACGGCTTT CCCTCCTGCT TGCGATGTCC   60
CTGTTAGTTA TGTGCGTCGC ATCCGTCGTA TCCCCTCCCC CGCAAGCGCT AGCATCAGGC  120
TCTGGCGGCA TCGAGCGGGT ATTCACCGAC AAAGCCCGCT ATAATCCAGG CGACGCTGTC  180
TCGATCCGCG TGCAGGCAAA AAACGGCACC GGCTCAAGCT GGAGCGGCGC CGCCCGACTG  240

GAAATTTTTC ATTTGGAGAA CTCGGTGTAC ACCTCTTCCC AATCCTTATC GTTAACAAAC 300
GGGCAATCCA CGACGCTGAC CTTCACTTGG ACGGCTCCAT CCACGGACTT CAGAGGTTAC 360
TTCGTCCGGA TCGATGCCGG GACGCTGGGG CAAGGAGCAA CGGCGATCGA CGTCTCCAGC 420
GACTTCACGA AATATCCGCG ATACGGATAT ATTAGCGAAT TCGAGTCCGG CGAGACGGCA 480
TTGGAGAGCA AGGCCAAGGT GGATCAGCTT GCGCAGGATT ACCACATTAA CGCCTGGCAG 540
TTCTACGACT GGATGTGGCG ACACGACAAA ATGATCAAGC GCACCGGCGG CAGCATCGAC 600
TCCACCTGGC TGGACTTGTT CAACCGCGAG ATTTCCTGGT CCACCCTTCA GAATCAGATC 660
GACGCGGTGC ATGACGTAAA CGGCAAGGCG ATGGCTTACG CCATGATCTA CGCCTCCCGC 720
GAGAACTATT CGCCTCTCGG CATCAGTCCG ACCTGGGGCA TCTACGAGGA CTCCTCCCAT 780
ACGAACCAGT TCGACGTCGA CTTCGGTGAC GGCAGCACGT ACCTCTATAT GTCCGACCCG 840
CAAAATCCCA ATTGGCAAAA TTATATCCAT GCCGAATACA TCGATTCCAT CAACACCGCG 900
GGCTTCGACG GCATCCACGT GGACCAGATG GGCCAGCGCA GCAACGTGTA CGATTACAAC 960
GGCAACTCCA TTGATCTCTC CACACGCTTC TCGCCGTTTC TGGACCAGGC CAAAAGCGTA 1020
CTTAGCGCTA ACAACCCTGC CCGCGACAAC CTGACCTATA ATATCGTCGA CGGCACGGTA 1080
AACGGATGGG CGGTAAACGA TGTAAGCAAA AACGCCGATC TCGACTTCCT GTACAGCGAA 1140
ATCTGGTATC TCTCGGACAG CTACAACCAG CTTAAAAACT ATATCGAGCA GCTACGCGCG 1200
AACGGCGGCA ACAAAGCGGT CGTGCTGGCA GCCTATATGA ACTATGCTGA CAATGCCGGC 1260
ACGCGTTACG AAGCGGAGAG CGCGTCGATG ACGAACGTCT CGACGAATAC GAATCATGCC 1320
GGGTACACGG GGAGCGGATT TGTCGACCAG TTCGCAAGCA CAGGCGATAA GGTTTCCTTT 1380
GCGATCAACG CTCCCGAGGC AGGAGATTAT TCGCTCGTAT TCCGTTACGG CAACAACACA 1440
GGAGCCAATT CGACGCTTAA TCTGTACGTG GACGGAAACT TCGTGCAGAA GCTGTATTTC 1500
TTCAACCAGA GCAGTTGGGG AACATGGAAG CACGACGCCT GGTACCAGGT GCCGCTGACG 1560
CAAGGAGCCC ACACCGTAGA GCTGCGGTAC GAATCCGGCA ACGTCGGCGC GGTTAACCTC 1620
GACAGCTTGA CGCTTGGCAC GTTCGACGAA CATTCCGTAC GGCTGGCGGA TGCCATGATG 1680
TCGGCGAGCG GCGCGACGCA TATCGAGCTG GGCGACGACA ACCAGATGCT GCCGCACGAA 1740
TATTATCCAA ACCGAAGCAA GACGATGCGA AGCTCGCTCA AAAACGCGAT GAAGGATCAT 1800
TACAACTTCA TCACGGCTTA CGAGAACCTG CTGTTCGACT CGGATGTCGT CCCTAACGAC 1860
ACCGGCTCGC AATTCGTGAA TCTGACGGGC GTCAGCGCAA GCGGCGACGG ATCGGCCAAC 1920

ACGGTCTGGT ATATCAATAA AAGGACGTCG GATTACAATA TCGTTCACCT CATTAATCTG 1980
CTCGGCAATG ATAACCAATG GCGCAATACG GCGTCGCAGC CAAGCTTCCA GACCAATCTT 2040
CCGGCTAAAA TCTACATCGG CGCGGACGAA ACGATTTCGG ACGTCTATCT CGCCTCGCCT 2100
GACCTGAGCG GCGGGGAGAC GCAGGAACTG GCATTCACTT CGGGCACGGA TGCCGGAGGC 2160
AAATACGTTT CCTTTACGGT GCCGGAGCTG AAGTACTGGA ATATGATCTA CATGAAACGT 2220
ACGTTCAGCG TGCCTGCTAA CGACATCTAT GAAGCGGAGA CGGCGATCAA GTCGAACGTC 2280
TCGACGAACA CGAATCATGC CGGCTATACG GGGAGCGGCT TCGTCGACGG ATTTTCTTCT 2340
ACAAATGACG GCGTCTCCTT CGTCGTGAAG TCGACGGCCT CCGACGATTA CGCGCTGCGT 2400
TTCCGTTATG CCAACGGCGG CTCCGACGCA ACCCGGGACG TGTACGTTGA TGGCAAGCTC 2460
GCAGGCACCG TTTCGTTTAA GAGCACGGGC TCTTGGAGCA CATGGAGCTA CGGCGAGATC 2520
ACCGCGCGTC TGGAGCCCGG CCACCACACC ATTGTGCTAT GGCAGACGAG CGGCAACACC 2580
GGGGCAATCA ACCTCGATCA TCTGGATCTC GACAAAACGT ACATTTGGCA GTTCGACCGT 2640
CAAATTGTCA GCGTACCTGC CGGATACCGC ATTACCTTCC GAACGGGATT GCCGGGCTGG 2700
GTGCACTGGG GCGTAAACGG CTGGACGGGC GTGACCGATA CGCCGCTCCG GAGCAACGGC 2760
TCCCTGGACG GCAATCTGGA TCACGAGACG TCCATCGGAC CGTTCGCCAC CGGCACCGCG 2820
GTCGACGTTA CGTTCCTGTG GGACGACAAC AACAACGGTA TCCTGGAACC AAGCACGGAC 2880
AGATGGGAAG GGACGGACTT CGGGATCAAT GTGAGC 2916

Sequence No: 3
Length: 24
Sequence Type: amino acid
Topology: linear
Nature: peptide
Sequence:
Ser Gly Ser Gly Gly Ile Glu Arg Val Phe Thr Asp Lys Ala Arg Tyr
                5                   10                  15
Asn Pro Gly Asp Ala Val Ser Ile
            20

Sequence No: 4

Length: 934

Sequence Type: amino acid

Topology: linear

Nature: peptide

Sequence:

Ser Gly Ser Gly Gly Ile Glu Arg Val Phe Thr Asp Lys Ala Arg Tyr

                5                   10                  15

Asn Pro Gly Asp Ala Val Ser Ile Arg Val Gln Ala Lys Asn Gly Thr

            20                  25                  30

Gly Ser Ser Trp Ser Gly Ala Ala Arg Leu Glu Ile Phe His Leu Glu

        35                  40                  45

Asn Ser Val Tyr Thr Ser Ser Gln Ser Leu Ser Leu Thr Asn Gly Gln

    50                  55                  60

Ser Thr Thr Leu Thr Phe Thr Trp Thr Ala Pro Ser Thr Asp Phe Arg

65                  70                  75                  80

Gly Tyr Phe Val Arg Ile Asp Ala Gly Thr Leu Gly Gln Gly Ala Thr

                85                  90                  95

Ala Ile Asp Val Ser Ser Asp Phe Thr Lys Tyr Pro Arg Tyr Gly Tyr

            100                 105                 110

Ile Ser Glu Phe Glu Ser Gly Glu Thr Ala Leu Glu Ser Lys Ala Lys

        115                 120                 125

Val Asp Gln Leu Ala Gln Asp Tyr His Ile Asn Ala Trp Gln Phe Tyr

    130                 135                 140

Asp Trp Met Trp Arg His Asp Lys Met Ile Lys Arg Thr Gly Gly Ser

145                 150                 155                 160

Ile Asp Ser Thr Trp Leu Asp Leu Phe Asn Arg Glu Ile Ser Trp Ser

                165                 170                 175

Thr Leu Gln Asn Gln Ile Asp Ala Val His Asp Val Asn Gly Lys Ala
            180                 185                 190
Met Ala Tyr Ala Met Ile Tyr Ala Ser Arg Glu Asn Tyr Ser Pro Leu
        195                 200                 205
Gly Ile Ser Pro Thr Trp Gly Ile Tyr Glu Asp Ser Ser His Thr Asn
    210                 215                 220
Gln Phe Asp Val Asp Phe Gly Asp Gly Ser Thr Tyr Leu Tyr Met Ser
225                 230                 235                 240
Asp Pro Gln Asn Pro Asn Trp Gln Asn Tyr Ile His Ala Glu Tyr Ile
                245                 250                 255
Asp Ser Ile Asn Thr Ala Gly Phe Asp Gly Ile His Val Asp Gln Met
            260                 265                 270
Gly Gln Arg Ser Asn Val Tyr Asp Tyr Asn Gly Asn Ser Ile Asp Leu
        275                 280                 285
Ser Thr Arg Phe Ser Pro Phe Leu Asp Gln Ala Lys Ser Val Leu Ser
    290                 295                 300
Ala Asn Asn Pro Ala Arg Asp Asn Leu Thr Tyr Asn Ile Val Asp Gly
305                 310                 315                 320
Thr Val Asn Gly Trp Ala Val Asn Asp Val Ser Lys Asn Ala Asp Leu
                325                 330                 335
Asp Phe Leu Tyr Ser Glu Ile Trp Tyr Leu Ser Asp Ser Tyr Asn Gln
            340                 345                 350
Leu Lys Asn Tyr Ile Glu Gln Leu Arg Ala Asn Gly Gly Asn Lys Ala
        355                 360                 365
Val Val Leu Ala Ala Tyr Met Asn Tyr Ala Asp Asn Ala Gly Thr Arg
    370                 375                 380
Tyr Glu Ala Glu Ser Ala Ser Met Thr Asn Val Ser Thr Asn Thr Asn
385                 390                 395                 400

His Ala Gly Tyr Thr Gly Ser Gly Phe Val Asp Gln Phe Ala Ser Thr
                405                 410                 415
Gly Asp Lys Val Ser Phe Ala Ile Asn Ala Pro Glu Ala Gly Asp Tyr
            420                 425                 430
Ser Leu Val Phe Arg Tyr Gly Asn Asn Thr Gly Ala Asn Ser Thr Leu
        435                 440                 445
Asn Leu Tyr Val Asp Gly Asn Phe Val Gln Lys Leu Tyr Phe Phe Asn
    450                 455                 460
Gln Ser Ser Trp Gly Thr Trp Lys His Asp Ala Trp Tyr Gln Val Pro
465                 470                 475                 480
Leu Thr Gln Gly Ala His Thr Val Glu Leu Arg Tyr Glu Ser Gly Asn
                485                 490                 495
Val Gly Ala Val Asn Leu Asp Ser Leu Thr Leu Gly Thr Phe Asp Glu
            500                 505                 510
His Ser Val Arg Leu Ala Asp Ala Met Met Ser Ala Ser Gly Ala Thr
        515                 520                 525
His Ile Glu Leu Gly Asp Asp Asn Gln Met Leu Pro His Glu Tyr Tyr
    530                 535                 540
Pro Asn Arg Ser Lys Thr Met Arg Ser Ser Leu Lys Asn Ala Met Lys
545                 550                 555                 560
Asp His Tyr Asn Phe Ile Thr Ala Tyr Glu Asn Leu Leu Phe Asp Ser
                565                 570                 575
Asp Val Val Pro Asn Asp Thr Gly Ser Gln Phe Val Asn Leu Thr Gly
            580                 585                 590
Val Ser Ala Ser Gly Asp Gly Ser Ala Asn Thr Val Trp Tyr Ile Asn
        595                 600                 605
Lys Arg Thr Ser Asp Tyr Asn Ile Val His Leu Ile Asn Leu Leu Gly
    610                 615                 620

Asn Asp Asn Gln Trp Arg Asn Thr Ala Ser Gln Pro Ser Phe Gln Thr
625                 630                 635                 640
Asn Leu Pro Ala Lys Ile Tyr Ile Gly Ala Asp Glu Thr Ile Ser Asp
                645                 650                 655
Val Tyr Leu Ala Ser Pro Asp Leu Ser Gly Gly Glu Thr Gln Glu Leu
            660                 665                 670
Ala Phe Thr Ser Gly Thr Asp Ala Gly Gly Lys Tyr Val Ser Phe Thr
        675                 680                 685
Val Pro Glu Leu Lys Tyr Trp Asn Met Ile Tyr Met Lys Arg Thr Phe
    690                 695                 700
Ser Val Pro Ala Asn Asp Ile Tyr Glu Ala Glu Thr Ala Ile Lys Ser
705                 710                 715                 720
Asn Val Ser Thr Asn Thr Asn His Ala Gly Tyr Thr Gly Ser Gly Phe
                725                 730                 735
Val Asp Gly Phe Ser Ser Thr Asn Asp Gly Val Ser Phe Val Val Lys
            740                 745                 750
Ser Thr Ala Ser Asp Asp Tyr Ala Leu Arg Phe Arg Tyr Ala Asn Gly
        755                 760                 765
Gly Ser Asp Ala Thr Arg Asp Val Tyr Val Asp Gly Lys Leu Ala Gly
    770                 775                 780
Thr Val Ser Phe Lys Ser Thr Gly Ser Trp Ser Thr Trp Ser Tyr Gly
785                 790                 795                 800
Glu Ile Thr Ala Arg Leu Glu Pro Gly His His Thr Ile Val Leu Trp
                805                 810                 815
Gln Thr Ser Gly Asn Thr Gly Ala Ile Asn Leu Asp His Leu Asp Leu
            820                 825                 830
Asp Lys Thr Tyr Ile Trp Gln Phe Asp Arg Gln Ile Val Ser Val Pro
        835                 840                 845

Ala Gly Tyr Arg Ile Thr Phe Arg Thr Gly Leu Pro Gly Trp Val His
    850                 855                 860
Trp Gly Val Asn Gly Trp Thr Gly Val Thr Asp Thr Pro Leu Arg Ser
865                 870                 875                 880
Asn Gly Ser Leu Asp Gly Asn Leu Asp His Glu Thr Ser Ile Gly Pro
                885                 890                 895
Phe Ala Thr Gly Thr Ala Val Asp Val Thr Phe Leu Trp Asp Asp Asn
            900                 905                 910
Asn Asn Gly Ile Leu Glu Pro Ser Thr Asp Arg Trp Glu Gly Thr Asp
        915                 920                 925
Phe Gly Ile Asn Val Ser
    930

## Claims

1. A DNA molecule encoding a protein having the enzymatic activity of a cycloisomaltooligosaccharide synthase selected from the group consisting of:
(a) DNA molecules having the restriction map 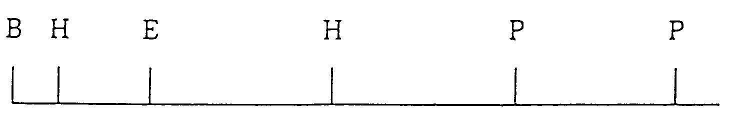
B H E H P P
 wherein B stands for BamHI, E for EcoRI, H for HindIII and P for PstI;
(b) DNA molecules coding for a protein having the amino acid sequence given in Sequence No. 1;
(c) DNA molecules coding for a protein having the amino acid sequence given in Sequence No. 4;
(d) DNA molecules having the nucleotide sequence given in Sequence No. 2;
(e) DNA molecules hybridising to a DNA molecule as defined in (a), (b), (c) or (d); and
(f) DNA molecules which have a DNA sequence which is degenerated as a result of the genetic code to the DNA sequences of DNA molecules as defined in (a) to (e).

2. The DNA molecule of claim 1, wherein the encoded cycloisomaltooligosaccharide synthase has a molecular weight of approximately 103 kD.

3. The DNA molecule of claim 1 or 2 which is derived from bacteria of the genus Bacillus.

4. A vector comprising a DNA molecule of any one of claims 1 to 3.

5. The vector of claim 4, in which the DNA molecule is combined with regulatory DNA elements ensuring expression in procaryotic or eucaryotic cells.

6. A host cell comprising a vector of claim 5.

7. The host cell of claim 6, which is Escherichia coli.

8. A process for producing cycloisomaltooligosaccharide synthase, which comprises culturing a host cell of claim 6 or 7 in a medium and recovering cycloisomaltooligosaccharide synthase from the culture.

9. A protein which is encoded by a DNA molecule of any one of claims 1 to 3.

10. Use of the protein of claim 9 for the production of cycloisomaltooligosaccharides.

11. Cycloisomaltooligosaccharides obtainable by the conversion of α-1,6-glucans into cyclisomaltooligosaccharides with the help of the protein of claim 9.
